## Europäisches Patentamt
## European Patent Office
## Office européen des brevets

(19)

(11) Numéro de publication: **0 148 072**
**B1**

(12) **FASCICULE DE BREVET EUROPÉEN**

(45) Date de publication du fascicule du brevet:
22.07.87

(51) Int. Cl.⁴: **A 23 J 3/00, C 12 P 21/06**

(21) Numéro de dépôt: **84402641.9**

(22) Date de dépôt: **18.12.84**

(54) **Procédé de protéolyse de protéines plasmatiques et produits obtenus par ce procédé.**

(30) Priorité: **29.12.83 FR 8321033**

(43) Date de publication de la demande:
**10.07.85 Bulletin 85/28**

(45) Mention de la délivrance du brevet:
**22.07.87 Bulletin 87/30**

(84) Etats contractants désignés:
**AT BE CH DE GB IT LI LU NL SE**

(56) Documents cité:
**AU-B-446 927**

**CHEMICAL ABSTRACTS, vol. 88, 1978, page 374, réf. no. 119056e, Columbus, Ohio, US; A.P. MIKHALKO et al.: "Kinetics of enzymic degradation of blood lymphocyte nuclei in normal and leukemic cattle" CHEMICAL ABSTRACTS, vol. 82, no. 7, 31 mars 1975, page 403, réf. no. 84787r, Columbus, Ohio, US**

(73) Titulaire: **ELECTRICITE DE FRANCE Service National, 2, rue Louis Murat, F-75008 Paris (FR)**
Titulaire: **INSTITUT BIO- LIMOUSIN, 123 rue Albert Thomas, F-87060 Limoges Cedex (FR)**

(72) Inventeur: **Julien, Raymond, 65- 67 Avenue Baudin, F-87000 Limoges (FR)**
Inventeur: **Bressollier, Philippe, 19, rue Eusèbe Bombal, F-87100 Limoges (FR)**
Inventeur: **Daumas, Christian, 10 Avenue des Marronniers, F-77210 Avon (FR)**

(74) Mandataire: **Bressand, Georges, c/o CABINET LAVOIX 2 Place d'Estienne d'Orves, F-75441 Paris Cedex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 148 072 B1

## Description

La présente invention concerne un procédé de protéolyse de protéines plasmatiques en vue de fournir des hydrolysats protéiques.

On cherche actuellement à valoriser un certain nombre de produits qui étaient, jusqu'à présent, en grande partie rejetés.

Parmi ces produits le sang, sous-produit de l'abattage des animaux, présente une valeur potentielle importante par sa teneur élevée en protéines. Mais, il s'avère qu'actuellement seule une faible partie du sang est récupérée et utilisée pour des applications diverses.

La présente invention vise à fournir un nouveau procédé permettant de valoriser le sang.

La présente invention vise plus spécialement à fournir un procédé permettant de valoriser les protéines plasmatiques par hydrolyse.

Lorsqu'on cherche à hydrolyser des protéines, on se heurte toutefois souvent à une difficulté, à savoir la saveur désagréable de ces hydrolysats, ce qui est un inconvénient majeur dans la mesure où les hydrolysats de protéines trouvent leur application essentielle dans l'alimentation humaine et animale.

Cela est le cas, en particulier, lorsqu'on effectue une hydrolyse enzymatique. On observe alors fréquemment des saveurs amères.

La présente invention vise également à fournir un procédé permettant d'obtenir des hydrolysats ayant un goût acceptable.

La présente invention vise plus spécifiquement à fournir un procédé par hydrolyse enzymatique permettant d'obtenir des hydrolysats ayant un goût acceptable.

De plus, si l'hydrolyse enzymatique présente un intérêt en raison de sa douceur et de sa spécificité, les opérations discontinues entrainent une mauvaise utilisation de l'enzyme. En effet, l'enzyme est perdue à la fin de l'opération.

La présente invention vise donc également à fournir un procédé d'hydrolyse enzymatique permettant la production en continu d'hydrolysats de protéines plasmatiques.

La présente invention a pour objet un procédé de protéolyse de protéines plasmatiques, caractérisé en ce qu'on effectue la protéolyse de protéines plasmatiques par la thermolysine, en présence d'ions calcium dans un milieu réactionnel aqueux ayant un pH de 5 à 7 et à une température inférieure ou égale à 80° C.

On a découvert en effet que la thermolysine, qui est une enzyme connue pour sa stabilité thermique en présence d'ions calcium, convenait particulièrement bien pour la protéolyse des protéines plasmatiques et conduisait, dans les conditions indiquées, à des hydrolysats sans saveur amère.

La thermolysine est une métalloendopeptidase isolée d'un filtrat de culture de Bacillus Thermoproteolyticus rokko. Elle a une masse molaire de 37.500 (déterminée par équilibre de sédimentation). Elle contient 4 atomes $Ca^{+2}$ assez fortement liés par molécule et est utilisée en présence d'ions calcium. Une concentration en calcium représentant cent fois la concentration en thermolysine est généralement suffisante. Des concentrations plus élevées peuvent être utilisées, mais généralement n'apportent pas d'avantage particulier.

Le pH du milieu réactionnel peut être maintenu à la valeur souhaitée par l'utilisation d'un acide, d'une base ou d'un tampon. On ajuste de préférence le pH à une valeur d'environ 5,5 à 6,5. Le tampon peut être, en particulier, un tampon barbital/acétate de sodium M/7, pH 6. On doit évidemment éviter les tampons qui complexent le calcium.

Bien qu'il soit, en théorie, possible d'opérer à des températures allant jusqu'à 80° C, la thermolysine devient instable à long terme au-delà de 60° C lorsqu'on respecte les autres conditions du procédé. On opère avantageusement à une température de 50 à 60° C.

La concentration des protéines dans le milieu réactionnel est avantageusement de 10 à 120 g/l et, de préférence, de 30 à 50 g/l.

La concentration en enzyme (pure) peut être notamment de 0,2 à 5 % en poids par rapport au poids de protéines.

On a, en outre, découvert que le rendement de la protéolyse pouvait être nettement accru si on effectuait au préalable une dénaturation des protéines plasmatiques.

Cette dénaturation peut être effectuée notamment par traitement thermique (chauffage) des protéines à une température de 70 à 100° C, le pH étant maintenu à une valeur de 5 à 7. Le temps de dénaturation est d'autant plus court que la température est plus élevée. A 70° C, le temps de dénaturation peut être d'environ 30 min.

La présente invention a également pour objet un procédé de protéolyse en continu de protéines plasmatiques. Selon ce procédé, on ajoute en continu des protéines plasmatiques à un milieu réactionnel, contenant de la thermolysine en présence d'ions calcium, ayant un pH de 5 à 7 et une température inférieure à 80° C, ce milieu étant maintenu dans un système comportant une paroi d'ultrafiltration, et on récupère les protéines hydrolysées dans le perméat d'ultrafiltration.

La thermolysine est ainsi, en quelque sorte, séquestrée dans le système.

On a découvert de façon surprenante, d'une part, que l'enzyme possède ainsi une stabilité élevée et, d'autre part, que par l'utilisation de la gamme de température préférée (50 à 60° C) on évite pratiquement toute prolifération bactérienne, phénomène qui est fréquent et particulièrement génant dans la protéolyse enzymatique.

Le système peut être une simple cellule d'ultrafiltration qui est alimentée en continu sous pression par les protéines plasmatiques.

Toutefois, dans un mode préféré de réalisation du procédé, on ajoute en continu dans un réacteur des protéines plasmatiques au milieu réactionnel contenant l'enzyme, on fait passer en continu le mélange à travers un dispositif d'ultrafiltration retenant l'enzyme et les protéines plasmatiques, on récupère les protéines hydrolysées dans le perméat d'ultrafiltration et on recycle le rétentat vers le réacteur.

Dans ce mode de réalisation, on utilise avantageusement comme dispositif d'ultrafiltration un dispositif à fibres creuses.

Un tel dispositif comprend un ensemble de fibres creuses microporeuses recouvertes intérieurement d'une "peau" d'ultrafiltration.

Un tel dispositif est avantaeux dans la mesure où le rapport de la surface au volume est très grand, les membranes sont autosupportées et on limite les phénomènes de polarisation de concentration grâce au flux laminaire imposé à la surface de la membrane.

En pratique, on peut utiliser un dispositif retenant les molécules ayant une masse molaire supérieure à 10.000.

On a, en outre, trouvé que si on soumet l'hydrolysat à une seconde ultrafiltration sur une membrane ayant un seuil de rétention plus faible, il est possible de séparer un rétentat contenant des glycopeptides. On peut utiliser pour la seconde ultrafiltration une membrane retenant les molécules ayant une masse molaire supérieure à 1000.

Les essais suivants illustrent la présente invention.

## I - Essais en discontinu

a) Préparation du substrat

On utilise comme substrat pour l'hydrolyse du plasma bovin. Le plasma bovin est obtenu par centrifugation du sang total recueilli sous anticoagulant (citrate trisodique 2 H$_2$O à 4 g/l) à l'abattage.

L'opération est conduite en continu, le sang total sous anticoagulant, à la température d'abattage, passe par un tamis de protection, puis par gravité dans un séparateur Westfalia type BTA 35.

Sous l'effet de la force centrifuge, on obtient deux phases:
- une phase légère jaune: le plasma
- une phase lourde rouge: le cruor.

Le rendement de l'opération est de 65 % (vol/vol) pour le plasma et 35 % (vol/vol) pour le cruor.

Le plasma ainsi obtenu est immédiatement stocké à +4°C, afin de limiter la contamination bactérienne.

Les caractéristiques biochimiques du plasma bovin sont les suivantes:
- Teneur en protéines totales: 65 g. l$^{-1}$ dont 45 % d'albumines et 55 % de globulines
- Teneur en lipides totaux: 10,7 g.kg$^{-1}$
- Teneur en glucides : g.l$^{-1}$
- pH = 7,46

- Na$^+$ = 139 mEq/l
- K$^+$ = 4,3 m Eq/l
- Cl$^-$ = 100 mEq/l
- Concentration en citrate sodique 2 H$_2$O = 4 g.l$^{-1}$

b) Préparation de l'enzyme

On utilise de la thermolysine Sigma qui se trouve sous forme d'un lyophilisat contenant 25 % de sels de tampon acétate de sodium et de calcium.

On dissout le lyophilisat dans un tampon barbital-acétate de sodium M/7, pH = 6,- CaCl$_2$ 2.10$^{-3}$M.

c) Méthode de protéolyse (essai standard)

Dans 40 ml de tampon barbital-acétate de Na M/7 pH : 6, on dissout la masse de plasma nécessaire pour atteindre la concentration protéique désirée dans un volume final de 50 ml. La concentration protéique est contrôlée par la méthode Kjedhal. L'ajustement de pH est réalisé par addition de HCl 1 N ou NaOH 1 N. La solution de protéines ainsi préparée est mise sous agitation et équilibrée à la température choisie pour réaliser l'hydrolyse. Au temps 0 de la réaction, on ajoute la thermolysine dissoute dans 10 ml de tampon barbital-acétate de sodium M/7 pH : 6, ainsi que la quantité de CaCl$_2$ nécessaire pour atteindre la concentration finale désirée.

Le pH du milieu réactionnel est contrôlé et ajusté en permanence grâce au système pH stat Metrohm.

En fonction du temps de réaction, on effectue des prélèvements dans le milieu afin de contrôler l'hydrolyse.

d) Essais

On effectue, divers essais de protéolyse avec, d'une part, un plasma bovin natif et, d'autre part, un plasma bovin dénaturé par chauffage à 70°C pendant 30 min.

Les conditions sont les suivantes:
Concentration de la thermolysine: 5.10$^{-6}$ M.l$^{-1}$
T = 50°C
pH = 6
(Ca$^{2+}$) = 2.10$^{-3}$ M.l$^{-1}$

On a mesuré la vitesse de protéolyse pour différentes concentrations en substrat.

La vitesse de protéolyse est exprimée en µmole d'équivalent leucine formée par minute selon la méthode de Ricks et coll. (1977, FEBS 11th meeting Copenhagen, vol. 44, Sympossium A Pergamon Press p. 119-128).

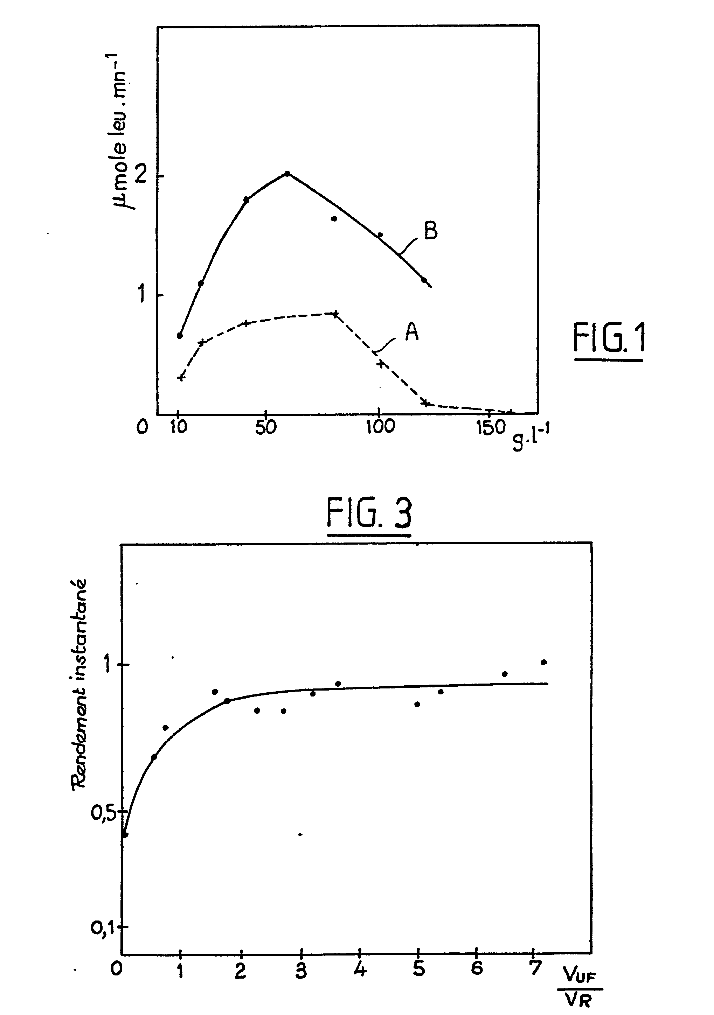

Les résultats sont reportés sur la Fig. 1 où la courbe A correspond au substrat natif et la courbe B au substrat thermodénaturé.

Ces courbes mettent en évidence un maximum en fonction de la concentration du substrat (de 80 g/l dans le cas du plasma natif et de 60 g/l dans le cas du plasma dénaturé). En outre, la comparaison de ces courbes montre que la thermodénaturation augmente très notablement la vitesse de protéolyse.

## II - Essais en continu

a) Installation d'essais

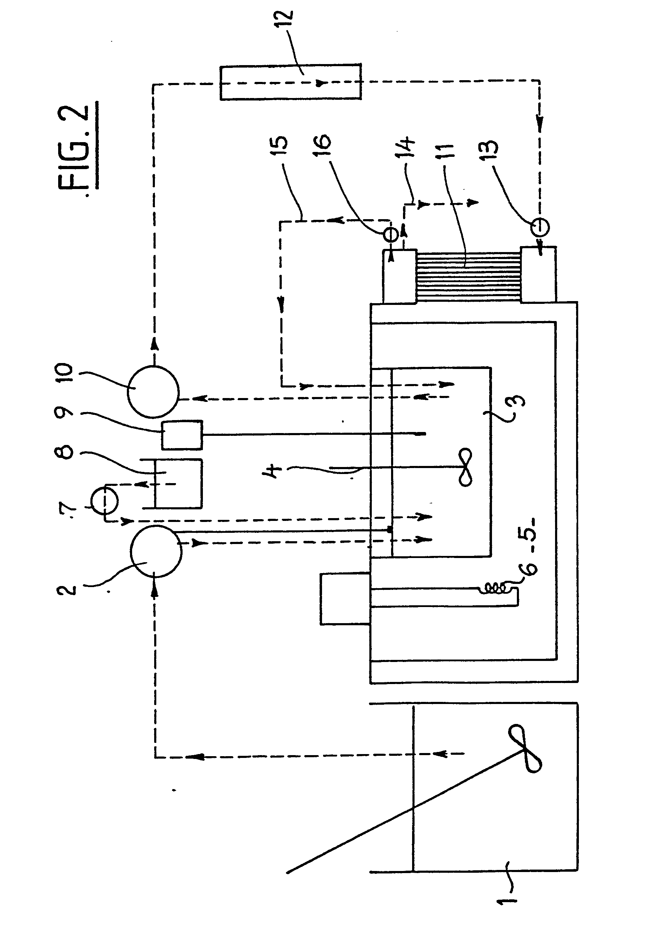

On a effectué les essais dans un réacteur enzymatique de laboratoire représenté schématiquement sur la Fig. 2.

Ce réacteur comprend un réservoir 1 contenant les protéines plasmatiques en milieu aqueux sous agitation.

Une pompe péristaltique 2 amène ces protéines dans le réacteur 3 proprement dit d'une capacité de 2 l. Ce réacteur est maintenu sous agitation par un agitateur 4.

Ce réacteur est constitué par une cuve plongeant dans un bain thermostaté 5 maintenu à la température appropriée par une résistance chauffante 6.

Une pompe 7 amène en outre une solution de $CaCl_2$ contenues dans un réservoir 8 dans le réacteur 3.

Le pH du milieu réactionnel contenu dans le réacteur 3 est ajusté en continu par un pH stat 9 (Metrhom E 526).

Une pompe 10 assure la circulation du milieu réactionnel vers un dispositif d'ultrafiltration 11 à fibres creuses en passant par un réfrigérant 12. Un manomètre 13 permet le contrôle de la pression d'entrée dans le dispositif d'ultrafiltration.

L'ultrafiltrat est évacué par un conduit 14, tandis que le rétentat est renvoyé par un conduit 15 muni d'une vanne de contrepression 16 vers le réacteur 3.

Le dispositif d'ultrafiltration à fibres creuses comprend une cartouche de fibres creuses de type Amicon $H_1P$ 10.8, en polysulfone, ayant un seuil de rétention de 10.000, une surface totale de 830 cm², avec un diamètre intérieur des fibres de 0,2 mm, une longueur de 20 cm, le nombre total de fibres étant de 1000.

b) Conditions expérimentales

Les conditions expérimentales sont les suivantes.

Le substrat est le plasma bovin dilué avec de l'eau et du NaCl 5%, contenant 30 g.l⁻¹ de protéines totales (mesurées par la méthode Kjeldhal) ajustées à pH 6 avec HCl 1 N, prédénaturées par chauffage préalable du substrat de pH 6 pendant 30 min. à 70°C. La thermolysine en solution dans 0,1 l de tampon barbital/acétate de sodium M/7 pH 6 est additionnée à 0,6 l de substrat maintenu à 60°C. Dans la cuve thermostatée du réacteur 3, la concentration finale en enzyme est de $10^{-5}$ M.l⁻¹

On fixe la concentration en calcium à $2.10^{-3}$ M.l⁻¹ (le calcium est ajouté en continu sous la forme d'une solution concentrée de $CaCl_2$ $2.10^{-2}$ M.l⁻¹, à l'aide de la pompe 7, dont le débit est fixé à 0,5 ml.mn⁻¹.

Après 30 min. d'incubation à 60°C, le milieu réactionnel est mis en recirculation dans le réacteur, le débit est fixé à 10 l.h⁻¹, le débit d'ultrafiltration, d, est égal à 0,3 l.h⁻¹. La température est maintenue à 60°C dans le réacteur, mais abaissée à 50°C dans le dispositif d'ultrafiltration.

c) Résultats

On a reporté sur la Fig. 3 le rendement instantané obtenu en fonction du rapport $\frac{VUF}{VR}$ étant le volume d'ultrafiltrat et VR le volume réactionnel.

On constate que ce rendement est élevé et stable sur une longue période.

Par ailleurs, on a constaté qu'il n'y avait pas accumulation des protéines hydrolysées dans le rétentat.

On a pu obtenir un fonctionnement continu correspondant à des valeurs $\frac{VUF}{VR}$ allant jusqu'à 13, c'est-à-dire un temps d'hydrolyse d'environ 30 heures.

Une filtration de l'hydrolysat sur Biogel $P_6$ met en évidence la présence de peptides ayant une masse molaire inférieure à 2000.

## III - Séparation d'une fraction glycopeptique à partir de l'hydrolysat

900 ml d'hydrolysats obtenus lors de la protéolyse en continu (comme indiqué en II) sont soumis à une ultrafiltration sur membrane $H_1P_1$ Amicon ayant un seuil de rétention de 1000 en utilisant un dispositif d'ultrafiltration à fibres creuses (dont la configuration est identique à celui utilisé en II).

On récupère dans le rétentat une fraction glycopeptique concentrée.

Ces glycopeptides sont dosés par la méthode de Dubois et coll. (Anal. Chem. 28, 3, p. 350-356).

L'hydrolysat obtenu selon la présente invention ou ses fractions peuvent trouver des applications, notamment dans l'industrie alimentaire humaine ou animale.

## Revendications

1. Procédé de protéolyse de protéines plasmatiques permettant d'obtenir des hydrolysats sans saveur amère, caractérisé en ce qu'on effectue la protéolyse des protéines plasmatiques par la thermolysine, en présence d'ions calcium, dans un milieu réactionnel aqueux ayant un pH de 5 à 7 et à une température inférieure ou égale à 80°C.

2. Procédé selon la revendication 1, caractérisé en ce qu'on effectue au préalable une dénaturation des protéines plasmatiques.

3. Procédé selon la revendication 2, caractérisé en ce qu'on effectue la dénaturation par chauffage à une température de 70 à 100°C.

4. Procédé selon l'une quelconque des revendications 1 à 3, caractérisé en ce qu'on règle la température du milieu réactionnel pendant la protéolyse à une valeur de 50 à 60°C.

5. Procédé selon l'une quelconque des revendications 1 à 3, caractérisé en ce qu'on règle le pH entre 5,5 et 6,5.

6. Procédé de protéolyse en continu de protéines plasmatiques permettant d'obtenir des hydrolysats sans saveur amère, caractérisé en ce qu'on ajoute en continu des protéines plasmatiques à un milieu réactionnel, contenant de la thermolysine en présence d'ions calcium, ayant un pH de 5 à 7 et une température inférieure à 80°C, ce milieu étant maintenu dans un système comportant une paroi d'ultrafiltration, et on récupère les protéines hydrolysées dans le perméat d'ultrafiltration.

7. Procédé selon la revendication 6, caractérisé en ce qu'on règle la température du milieu réactionnel à une valeur de 50 à 60°C.

8. Procédé selon la revendication 6 ou la revendication 7, caractérisé en ce qu'on effectue au préalable une dénaturation des protéines plasmatiques.

9. Procédé selon l'une quelconque des revendications 6 à 8, caractérisé en ce qu'on ajoute en continu dans un réacteur des protéines plasmatiques au milieu réactionnel contenant l'enzyme, on fait passer en continu le mélange à travers un dispositif d'ultrafiltration retenant l'enzyme et les protéines plasmatiques, on récupère les protéines hydrolysées dans le perméat d'ultrafiltration et on recycle le rétentat vers le réacteur.

10. Procédé selon la revendication 9, caractérisé en ce qu'on utilise comme dispositif d'ultrafiltration un dispositif à fibres creuses.

11. Procédé selon la revendication 9 ou la revendication 10, caractérisé en ce que le dispositif d'ultrafiltration retient les molécules de masse molaire supérieure à 10.000.

12. Procédé selon l'une quelconque des revendications précédentes, caractérisé en ce qu'on soumet l'hydrolysat à une seconde ultrafiltration de façon à obtenir un rétentat contenant des glycopeptides.

13. Procédé selon la revendication 12, caractérisé en ce qu'on utilise pour la seconde ultrafiltration une membrane retenant les molécules ayant une masse molaire supérieure à 1000.

14. Hydrolysat de protéines obtenu par un procédé selon l'une quelconque des revendications 1 à 11.

15. Glycopeptides obtenus par un procédé selon l'une quelconque des revendications 12 et 13.


**Patentansprüche**

1. Verfahren zur Proteolyse von Plasmaproteinen zur Herstellung von Hydrolysaten ohne bitteren Geschmack, dadurch gekennzeichnet, daß die Proteolyse der Plasmaproteine durch das Thermolysin in Gegenwart von Kalziumionen in einem wässrigen Reaktionsmedium mit einem pH-Wert von 5 bis 7 und einer Temperatur von 80°C oder weniger durchgeführt wird.

2. Verfahren nach Anspruch 1, dadurch gekennzeichnet, daß vorher eine Denaturierung der Plasmaproteine durchgeführt wird.

3. Verfahren nach Anspruch 2, dadurch gekennzeichnet, daß die Denaturierung durch Erhitzen auf eine Temperatur von 70 bis 100°C durchgeführt wird.

4. Verfahren nach einem der Ansprüche 1 bis 3, dadurch gekennzeichnet, daß die Temperatur des Reaktionsmediums während der Proteolyse auf einen Wert von 50 bis 60°C eingestellt wird.

5. Verfahren nach einem der Ansprüche 1 bis 3, dadurch gekennzeichnet, daß der pH-Wert zwischen 5,5 und 6,5 eingestellt wird.

6. Verfahren zur kontinuierlichen Proteolyse von Plasmaproteinen zur Herstellung von Hydrolysaten ohne bitteren Geschmack, dadurch gekennzeichnet , daß die Plasmaproteine kontinuierlich zu einem Reaktionsmedium, welches Thermolysin in Gegenwart von Kalziumionem enthält und dessen pH-Wert 5 bis 7 und Temperatur weniger als 80°C betragen, zugegeben werden, wobei das Medium einem aus einer Ultrafiltrationswand bestehenden System zugeführt wird und die hydrolysierten Proteine aus dem Ultrafiltrationsdurchlauf gewonnen werden.

7. Veriahren nach Anspruch 6, dadurch gekennzeichnet, daß die Temperatur des Reaktionamediums auf einen Wert von 50 bis 60°C eingestellt wird.

8. Verfahren nach den Ansprüchen 6 oder 7, dadurch gekennzeichnet, das vorher eine Denaturierung der Plasmaproteine durchgeführt wird.

9. Verfahren nach einem der Ansprüche 6 bis 8, dadurch gekennzeichnet, daß in einem Reaktor die Plasmaproteine in ein das Enzym enthaltendes Reaktionsmedium kontinuierlich zugegeben werden, die Mischung durch eine das Enzym und die Plasmaproteine rückhaltende Ultrafiltrationsvorrichtung geschickt, die hydrolysierten Proteine im Ultrafiltrationsdurchlauf gewonnen und der Filterrückstandsrest wieder in den Reaktor zurückgeführt werden.

10. Verfahren nach Anspruch 9, dadurch gekennzeichnet, daß als Ultrafiltrationsvorrichtung eine Vorrichtung aus Hohlfasern verwendet wird.

11. Verfahren nach den Ansprüchen 9 oder 10, dadurch gekennzeichnet , daß die ultrafiltrationsvorrichtung alle Moleküle mit einem Molekulargewicht von über 10.000 aufhält.

12. Verfahren nach einem der vorangegangen Ansprüche, dadurch gekennzeichnet, daß das Hydrolysat einer zweiten Ultrafiltration unterworfen wird unter Gewinnung eines glycopeptidhaltigen Filterrückstandes.

13. Verfahren nach Anspruch 12, dadurch gekennzeichnet, daß für die zweite Ultrafiltration eine Membran verwendet wird, die Moleküle mit einem Molekulargewicht über 1.000 aufhält.

14. Proteinhydrolysat, erhalten durch ein Verfahren nach einem der Ansprüche 1 bis 11.

15. Glycopeptide, erhalten durch ein Verfahren nach einem der Ansprüche 12 und 13.

## Claims

1. Process for proteolysis of plasma proteins which enables hydrolyzates without a bitter flavor to be obtained, characterized in that the proteolysis of the plasma proteins is performed by thermolysin in the presence of calcium ions in an aqueous reaction medium of pH 5 to 7 at a temperature below or equal to 80°C.

2. Process as claimed in claim 1, characterized in that prior denaturation of the plasma proteins is performed.

3. Process as claimed in claim 2, characterized in that the denaturation is accomplished by heating to a temperature of 70 to 100°C.

4. Process as claimed in any one of claims 1 to 3, characterized in that the temperature of the reaction medium is adjusted during the proteolysis to a value of 50 to 60°C.

5. Process as claimed in any one of claims 1 to 3, characterized in that the pH is adjusted to between 5.5 and 6.5.

6. Process for continuous proteolysis of plasma proteins which enable hydrolyzates without a bitter flavor to be obtained, characterized in that plasma proteins are added continuously to a reaction medium containing thermolysin in the presence of calcium ions at pH 5 to 7 and at a temperature below 80°C, this medium being maintained in a system which incorporates an ultrafiltration surface, and the hydrolyzed proteins are recovered in the ultrafiltration permeate.

7. Process as claimed in claim 6, characterized in that the temperature of the reaction medium is adjusted to a value of 50 to 60°C.

8. Process as claimed in claim 6 or claim 7, characterized in that prior denaturation of the plasma proteins is performed.

9. Process as claimed in any one of claims 6 to 8, characterized in that plasma proteins are added continuously to the reaction medium containing the enzyme in a reactor, the mixture is passed continuously through an ultrafiltration device which retains the enzyme and the plasma proteins, the hydrolyzed proteins are recovered in the ultrafiltration permeate and the retentate is recycled to the reactor.

10. Process as claimed in claim 9, characterized in that a hollow fiber device is used as ultrafiltration device.

11. Process as claimed in claim 9 or claim 10, characterized in that the ultrafiltration device retains the molecules of molar mass greater than 10,000.

12. Process as claimed in any one of the preceding claims, characterized in that the hydrolyzate is subjected to a second ultrafiltration so as to obtain a retentate containing glycopeptides.

13. Process as claimed in claim 12, characterized in that a membrane which retains molecules having a molar mass greater than 1000 is used for the second ultrafiltration.

14. Protein hydrolyzate obtained by a process as claimed in any one of claims 1 to 11.

15. Clycopeptides obtained by a process as claimed in either one of claims 12 and 13.

FIG.1

FIG. 3

FIG. 2

0 148 072